# FASCICULE DE BREVET EUROPEEN

(11) **EP 0 446 132 B1**
(45) Date de publication et mention de la délivrance du brevet: **14.09.1994**
(21) Numéro de dépôt: 91400618.4
(22) Date de dépôt: 06.03.1991
(51) Int. Cl.: A61K 7/13

(54) **Procédé de teinture des fibres kératiniques avec des 6 ou 7-monohydroxyindoles à pH acide et compositions**
Verfahren zur Färbung von keratinischen Fasern mit 6- oder 7-Monohydroxyindolen unter säurischem pH und Zusammensetzungen
Keratinous fibres dyeing process with 6 or 7-monohydroxyindols at acidic pH and compositions

(30) Priorité: 08.03.1990 FR 9002975
(43) Date de publication de la demande: 11.09.1991
(73) Titulaire: L'OREAL, 75008 Paris (FR)
(72) Inventeur: Cotteret, Jean, F-78480 Verneuil-sur-Seine (FR); Audousset, Marie Pascale, F-92300 Levallois-Perret (FR)
(74) Mandataire: Casalonga, Axel

(56) Documents cités:
- EP-A- 0 360 638
- FR-A- 2 626 771
- FR-A- 2 636 235
- FR-A- 2 636 236
- GB-A- 2 207 443
- GB-A- 2 211 517

## Description

La présente invention est relative à un nouveau procédé de teinture des fibres kératiniques, en particulier des fibres kératiniques humaines, mettant en oeuvre des 5,/6 ou 7-hydroxyindoles en association avec des bases d'oxydation et un agent oxydant en milieu acide et aux compositions mises en oeuvre au cours de ce procédé. D'après Ch. Zwiak "Science des traitements capillaires" 1988, (HASSON) pages 263 à 268, il est connu de teindre les fibres kératiniques et en particulier les cheveux humains avec des compositions tinctoriales contenant des précurseurs de colorants d'oxydation et en particulier des p-phénylènediamines, des ortho ou para-aminophénols appelés généralement "bases d'oxydation" et présentant un pH alcalin.

On sait également d'après ce même ouvrage que l'on peut faire varier les nuances obtenues avec ces bases d'oxydation en les associant à des coupleurs encore appelés modificateurs de coloration, choisis notamment parmi les métadiamines aromatiques, les méta-aminophénols et les métadiphénols.

On cherche généralement à obtenir des colorations ayant de bonnes résistances à la lumière, aux lavages, à la transpiration et aux intempéries, notamment par le choix de coupleurs et/ou de bases d'oxydation permettant d'obtenir de tels résultats.

La demanderesse vient de découvrir que l'utilisation de dérivés du 5,/6 et/ou du 7-monohydroxyindole associés à des bases d'oxydation lorsque cette association était appliquée en présence d'un agent oxydant et à pH acide sur les cheveux, conduisait à des colorations présentant une puissance tinctoriale améliorée. Les colorations ainsi obtenues présentent par ailleurs une excellente ténacité à la lumière, aux lavages, à la transpiration et aux intempéries.

Ces résultats sont particulièrement surprenants lorsque l'on compare ces résultats par rapport à ceux obtenus avec des coupleurs classiques de la série benzénique mentionnés ci-dessus où l'on constate souvent une perte de puissance tinctoriale et une ténacité plus faible, lorsqu'on opère à pH acide.

La présente invention a donc pour objet un procédé de teinture des fibres kératiniques, en particulier des fibres kératiniques humaines telles que les cheveux, comprenant l'application sur ces fibres d'au moins une composition contenant un dérivé de 5,/6 et/ou 7-monohydroxyindole, un précurseur de colorant d'oxydation encore appelé base d'oxydation et un agent oxydant, à pH acide.

L'invention a également pour objet un agent de teinture à deux composants, dont l'un des composants comprend le dérivé du 5,/6 ou 7-monohydroxyindole et le précurseur du colorant d'oxydation et l'autre l'agent oxydant.

L'invention a également pour objet la composition prête à l'emploi, contenant les différents agents utilisés pour la teinture en milieu acide des cheveux.

D'autres objets de l'invention apparaitront à la lecture de la description et des exemples qui suivent.

Le procédé de teinture des fibres kératiniques et en particulier des fibres kératiniques humaines telles que les cheveux, conforme à l'invention, est essentiellement caractérisé par le fait que l'on applique sur ces fibres au moins une composition contenant dans un milieu approprié pour la teinture, un coupleur indolique répondant à la formule : 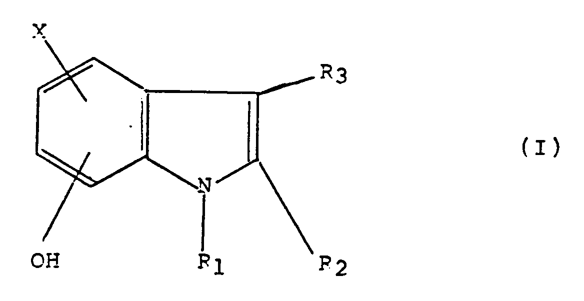
dans laquelle R₁ désigne un atome d'hydrogène, un radical alkyle en C₁-C₄; R₂ et R₃, identiques ou différents, désignent un atome d'hydrogène, un radical alkyle inférieur en C₁-C₄, un radical carboxyle ou un radical alcoxycarbonyle; X désigne un atone d'hydrogène, un radical alkyle inférieur en C₁-C₄, un radical alcoxy en C₁-C₁₈, un atome d'halogène, un radical acyloxy en C₂-C₂₀, un groupement acétylamino; le groupement OH occupant les positions 5 ou 6 ou 7 du cycle aromatique; ainsi que les sels de ces composés;
au moins un précurseur de colorant d'oxydation ou base d'oxydation;
au moins un agent oxydant; le pH de la composition appliqué sur les fibres étant inférieur à 7.

Les composés préférés répondant à la formule (I), utilisés conformément à l'invention, sont les composés dans lesquels le radical alkyle désigne de préférence méthyle, éthyle; le radical alcoxycarbonyle désigne méthoxy ou éthoxycarbonyle; le radical alcoxy désigne méthoxy, éthoxy, butoxy, hexadécyloxy; le radical acyloxy désigne acétoxy, tétradécanoyloxy.

Parmi ces composés, on peut citer le 6-nydroxyindole, le 6-hydroxy 3-méthoxycarbonylindole, le 6-hydroxy 1-méthyl 3-méthoxycarbonylindole, le 6-hydroxy 1-méthyl 2,3-diméthoxycarbonylindole, le 6-hydroxy 1,2-diméthylindole, le 6-hydroxy 2-méthyl indole, le 6-hydroxy 2-carboxyindole, le 6-hydroxy 2,3-diméthylindole, le 6-hydroxy 3-carboxyindole, le 6-hydroxy 3-éthoxycarbonylindole, le 6-hydroxy 2-éthoxycarbonylindole, le 6-hydroxy 3-méthylindole, le 6-hydroxy 1-méthylindole, le 6-hydroxy 5-acétoxyindole, le 6-hydroxy 5-méthoxyindole, le 6-hydroxy 5-méthoxy 2-méthylindole, le 6-hydroxy 5-butoxyindole, l'acide 6-hydroxy 5-méthoxyindole 2-carboxylique, le 6-hydroxy 5-méthoxy 2,3-diméthylindole, le 6-hydroxy 7-méthoxy indole, le 6-hydroxy 5-tétradécanoyloxyindole, le 6-hydroxy 5-hexadécyloxyindole, le 7-hydroxyindole, le 7-hydroxy 3-méthylindole, le 7-hydroxy 4-méthoxy 2,3-diméthylindole, le 6-hydroxy 5-méthoxy 1-méthylindole, le 6-hydroxy 7-méthylindole, le 6-hydroxy 2-éthoxycarbonyl 5-méthoxyindole, le 6-hydroxy 2-méthoxycarbonyl 5-butylindole, le 6-hydroxy 2,3-diméthyl-5-(acétylamino)indole, le 5-hydroxy 2-méthyl 3-éthoxy-carbonylindole, le 5-hydroxy 2-carboxyindole.

Les sels sont choisis plus particulièrement parmi les sels d'addition d'acides, tels que d'acide chlorhydrique, sulfurique, etc...

Les 5 ou 6 ou 7-hydroxyindoles plus particulièrement préférés sont choisis parmi le 6-hydroxyindole, le N-méthyl 6-hydroxyindole, le 2-carboxy 6-hydroxyindole, le N-méthyl 5-méthoxy 6-hydroxyindole, le 7-hydroxyindole, le 7-méthyl 6-hydroxyindole, le 2-méthyl 5-méthoxy 6-hydroxyindole, le 2,3-diméthyl 5-méthoxy 6-hydroxyindole, le 5-méthoxy 6-hydroxyindole, le 5-acétoxy 6-hydroxyindole, le 7-méthoxy 6-hydroxyindole, le 2-carboxy 5-méthoxy 6-hydroxyindole, le 3-méthyl 7-hydroxyindole, le 5-hydroxyindole, le 3-méthyl 5-hydroxyindole, le 2-éthoxycarbonyl 5-hydroxyindole, le 2,3-diméthyl 5-hydroxy 6-méthoxyindole, le 5-hydroxy 6-méthoxyindole.

Les précurseurs de colorants d'oxydation ou bases d'oxydation sont des composés connus en eux-mêmes qui ne sont pas des colorants en eux-mêmes et qui forment un colorant par un processus de condensation oxydative, soit sur eux-mêmes, soit en présence d'un coupleur ou modificateur. Ces composés comportent généralement un noyau aromatique portant des groupements fonctionnels, constitués : soit par deux groupements amino; soit par un groupement amino et un groupement hydroxy; ces groupements étant en position para ou ortho, l'un par rapport à l'autre.

Les précurseurs de colorants d'oxydation de type para, utilisés conformément à l'invention, sont choisis plus particulièrement parmi les paraphénylène diamines, les para-aminophénols, les précurseurs hétérocycliques para, comme la 2,5-diaminopyridine, la 2-hydroxy 5-aminopyridine, la 2,4,5,6-tétraamino pyrimidine.

Parmi les paraphénylènediamines, on peut citer plus particulièrement les composés répondant à la formule (II) : 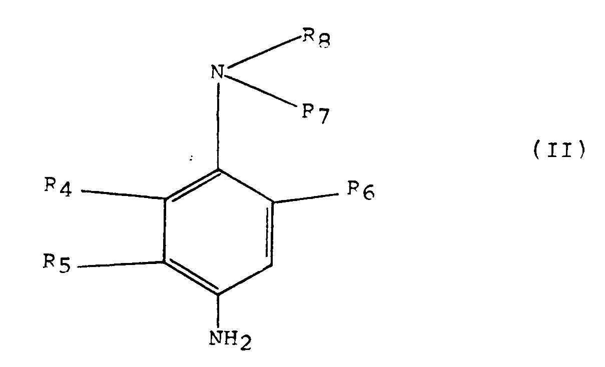
dans laquelle R₄, R₅ et R₆, identiques ou différents, représentent un atome d'hydrogène ou d'halogène, un radical alkyle ayant de 1 à 4 atomes de carbone, un radical alcoxy ayant de 1 à 4 atomes de carbone; R₇ et R₈, identiques ou différents, représentent un atome d'hydrogène, un radical alkyle, hydroxyalkyle, alcoxy alkyle, carbamylalkyle, mésylaminoalkyle, acétylamino alkyle, uréidoalkyle, carbalcoxyaminoalkyle, pipéridino alkyle, morpholinoalkyle; ces groupes alkyle ou alcoxy ayant de 1 à 4 atomes de carbone, ou bien R₇ et R₈ forment, conjointement avec l'atome d'azote auquel ils sont liés, un hétérocycle pipéridino ou morpholino, sous réserve que R₄ ou R₆ représente un atome d'hydrogène lorsque R₇ et R₈ ne représentent pas un atome d'hydrogène, ainsi que les sels de ces composés.

Parmi les composés particulièrement préférés répondant à la formule (II), on peut citer la p-phénylènediamine, la p-toluylènediamine, la méthoxy paraphénylènediamine, la chloroparaphénylènediamine, la 2,6-diméthylparaphénylènediamine, la 2,5-diméthylpara phénylènediamine, la 2,3-diméthylparaphénylènediamine, la 2-méthyl 5-méthoxyparaphénylènediamine, la 2,6-diméthyl 5-méthoxyparaphénylènediamine, la N,N-diméthyl paraphénylènediamine, la 3-méthyl 4-amino N,N-diéthyl aniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4′-amino)phényl] morpholine, la N-[(4′-amino)phényl] pipéridine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl) 4-aminophénol, le 2-méthoxy 4-aminophénol, le 3-méthoxy 4-aminophénol, le 2,5-diméthyl 4-aminophénol, le 2-méthoxyméthyl 4-aminophénol.

Les précurseurs de colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène et les orthophénylènediamines.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

Le pH de la composition appliqué sur les fibres kératiniques, en particulier les cheveux, a une valeur inférieure à 7 et est compris de préférence entre 3 et 6,9. Ce pH est ajusté par l'utilisation d'agents acidifiants bien connus dans le domaine de la teinture des fibres kératiniques, et en particulier des cheveux humains, tels que des acides minéraux organiques comme l'acide chlorhydrique, l'acide tartrique, l'acide citrique, l'acide phosphorique, les acides carboxyliques ou sulfoniques.

Les composés de formule (I) de la famille des 6 ou 7-hydroxyindoles sont présents dans la composition appliquée sur les fibres kératiniques, dans des proportions comprises de préférence entre 0,01 et 3,5% en poids par rapport au poids total de la composition.

Les compositions définies ci-dessus appliquées dans la teinture des fibres kératiniques peuvent également contenir en plus des coupleurs hétérocycliques de la famille des 6 ou 7-hydroxyindoles de formule (I), d'autres coupleurs connus en eux-mêmes tels que des métadiphénols, des métaaminophénols, des métaphénylène diamines, des métaacylaminophénols, les métauréido phénols, des métacarbalcoxyaminophénols, l'α-naphtol, des coupleurs possédant un groupement méthylène actif, tels que les composés β-cétoniques, les pyrazolones.

Parmi ces coupleurs pouvant être utilisés en plus des coupleurs de la famille des 6 ou 7-dihydroxy indoles de formule (I), on peut plus particulièrement citer le 2,4-dihydroxyphénoxyéthanol, le 2,4-dihydroxy anisole, le métaaminophénol, la résorcine, le mono méthyléther de résorcine, la 2-méthylrésorcine, le 2-méthyl 5-N-(β-hydroxyéthyl)aminophénol, le 2-méthyl 5-N-(β-mésylaminoéthyl)aminophénol, la 6-hydroxybenzomorpholine, le 2,4-diaminoanisole, le 2,4-diamino phénoxyéthanol, la 6-aminobenzomorpholine, le [2-N-(β-hydroxyéthyl)amino 4-amino]-phénoxyéthanol, le 2-amino 4-N-(β-hydroxyéthyl)aminoanisole, le (2,4-diamino)phényl-β, γ-dihydroxypropyléther, la 2,4-diaminophénoxyéthylamine, le 2-méthyl 5-aminophénol, le 2,6-diméthyl 3-aminophénol, le 3,4-méthylènedioxyphénol, le 3,4-méthylènedioxyaniline et leurs sels.

Ces compositions peuvent également contenir des agents tensio-actifs anioniques, cationiques, non ioniques, amphotères ou leurs mélanges.

Parmi ces agents tensio-actifs, on peut citer les alkylbenzènesulfonates, les alkylnaphtalène sulfonates, les sulfates, les éthersulfates et les sulfonates d'alcools gras, les sels d'ammonium quaternaires tels que le bromure de triméthylcétyl ammonium, le bromure de cétylpyridinium, les éthanolamides d'acides gras éventuellement oxyéthylénés, les acides, les alcools ou les amines polyoxyéthylénés, les alcools polyglycérolés, les alkylphénols polyoxyéthylénés ou polyglycérolés, ainsi que les alkylsulfates polyoxyéthylénés.

Les compositions tinctoriales sont généralement aqueuses, mais elles peuvent également contenir des solvants organiques pour solubiliser des composés qui ne seraient pas suffisamment solubles dans l'eau. Parmi ces solvants, on peut citer à titre d'exemple, les alcanols inférieurs en C₂-C₄ tels que l'éthanol et l'isopropanol, le glycérol, les glycols ou éthers de glycol, comme le butoxy-2 éthanol, l'éthylène glycol, le propylèneglycol, le monoéthyléther et le monométhyléther du diéthylèneglycol, ainsi que les alcools aromatiques comme l'alcool benzylique ou le phénoxyéthanol, ou les mélanges de ces solvants.

La composition appliquée sur les cheveux peut également renfermer des agents épaississants choisis en particulier parmi l'alginate de sodium, la gomme arabique, les dérivés de cellulose tels que la méthylcellulose, l'hydroxyéthylcellulose, l'hydroxy propylcellulose, l'hydroxyméthylcellulose, la carboxy méthylcellulose, les polymères d'acide acrylique éventuellement réticulés, la gomme de xanthane. On peut également utiliser des agents épaississants minéraux tels que la bentonite.

La composition peut égalememt renfermer les agents antioxydants choisis en particulier parmi le sulfite de sodium, l'acide thioglycolique, le bisulfite de sodium, l'acide ascorbique et l'hydroquinone, ainsi que d'autres adjuvants cosmétiquement acceptables lorsque la composition est destinée à être utilisée pour la teinture des fibres kératiniques humaines, tels que des agents de pénétration, des agents séquestrants, des conservateurs, des tampons, des parfums, etc...

Les compositions mises en oeuvre dans le procédé ne contiennent ni ion iodure, ni ion nitrite en quantité suffisante pour oxyder le coupleur de formule (I) et la base d'oxydation.

La composition appliquée sur les cheveux peut se présenter sous des formes diverses, telles que de liquides, de crèmes, de gels ou sous toute autre forme appropriée pour réaliser une teinture capillaire. Elle peut être conditionnée en flacon aérosol en présence d'un agent propulseur.

L'invention a également pour objet la composition prête à l'emploi utilisée dans le procédé défini ci-dessus.

Selon une forme de réalisation particulièrement préférée, le procédé comporte une étape préliminaire consistant à stocker sous forme séparée, d'une part, la composition contenant dans un milieu approprié pour la teinture le coupleur indolique répondant à la formule (I) définie ci-dessus et les précurseurs de colorants par oxydation sous forme d'un composant (A) et, d'autre part, une composition renfermant l'agent oxydant tel que défini ci-dessus sous forme d'un composant (B), et à procéder à leur mélange extemporané avant d'appliquer ce mélange sur les fibres kératiniques, comme indiqué ci-dessus.

La composition appliquée sur les fibres kératiniques résulte en particulier d'un mélange de 10 à 90% du composant (A) avec 90 à 10% du composant (B) contenant un agent oxydant.

L'invention a également pour objet un agent de teinture des fibres kératiniques, en particulier des cheveux, essentiellement caractérisé par le fait qu'il comporte au moins deux composants, l'un des composants étant constitué par le composant (A) défini ci-dessus et l'autre étant constitué par le composant (B) également défini ci-dessus, le pH des composants (A) et (B) étant tel qu'après mélange dans des proportions de 90 à 10% pour le composant (A) et de 10 à 90% pour le composant (B), la composition résultante ait un pH inférieur à 7.

Dans cette forme de réalisation, le composant (A) qui renferme au moins le dérivé indolique de la famille des 6 ou 7-hydroxyindoles de formule (I) et un précurseur de colorant d'oxydation, peut avoir un pH compris entre 3 et 10,5 et peut être ajusté à la valeur choisie au moyen d'agents alcalinisants habituellement utilisés en teinture des fibres kératiniques, tels que l'ammoniaque, les carbonates alcalins, les alcanolamines tels que les mono-, di- et triéthanolamines ainsi que leurs dérivés ou des agents acidifiants classiques, tels que les acides minéraux ou organiques, tels que les acides chlorhydrique, tartrique, citrique, phosphorique.

Cette composition peut renfermer les différents autres adjuvants mentionnés ci-dessus, notamment des coupleurs différents des coupleurs de la famille des 6 ou 7-hydroxyindoles répondant à la formule (I) déjà mentionnée ci-dessus.

L'ensemble des précurseurs de colorants par oxydation de type para et/ou ortho ainsi que les coupleurs, sont présents dans des proportions comprises de préférence entre 0,3 et 7% en poids par rapport au poids total du composant (A). La concentration en composés de formule (I) peut varier entre 0,05 et 4% en poids par rapport au poids total du composant A.

Les agents tensio-actifs sont présents dans le composant (A) dans des proportions de 0,1 à 55% en poids. Les agents solvants éventuellement présents en plus de l'eau sont présents dans des proportions comprises entre 0,5 et 40% en poids et en particulier entre 5 et 30% en poids par rapport au poids total du composant (A). Les agents épaississants sont présents de préférence dans des proportions comprises entre 0,1 et 5%, et en particulier entre 0,2 et 3% en poids. Les agents antioxydants mentionnés ci-dessus sont de préférence présents dans le composant (A) dans des proportions comprises entre 0,02 et 1,5% en poids par rapport au poids total du composant (A).

Le composant (B) renfermant l'agent oxydant tel que défini ci-dessus, a un pH inférieur à 7. Ce pH peut avoir une valeur minimum de 1 et de préférence il est compris entre 1,5 et 3,5. Ce composant (B) peut être acidifié avec le même type d'agents acidifiants que ceux utilisés pour le composant (A).

Il peut se présenter sous forme de liquides plus ou moins épaissis, de lait ou de gel.

Cet agent de teinture à deux composants peut être conditionné dans un dispositif à plusieurs compartiments ou kit de teinture, ou tout autre système de conditionnement à plusieurs compartiments dont l'un des compartiments renferme le composant (A) et le second compartiment renferme le composant (B); ces dispositifs pouvant être équipés d'un moyen permettant de délivrer sur les cheveux le mélange souhaité, tel que les dispositifs décrits plus particulièrement dans le brevet US-A-4 823 985 de la demanderesse.

L'invention a également pour objet l'utilisation comme coupleur de dérivés de 6 ou 7-hydroxyindoles répondant à la formule (I) pour la teinture un milieu acide des fibres kératiniques, en association avec des précurseurs de colorants d'oxydation.

Conformément à l'invention, le procédé de teinture consiste à appliquer sur les cheveux le mélange obtenu, à le laisser poser pendant 3 à 40 minutes, puis à rincer les cheveux et éventuellement effectuer un shampooing.

Il est également possible, conformément à l'invention, d'appliquer séparément une composition contenant le coupleur indolique de formule (I), le précurseur de colorant d'oxydation et l'agent oxydant, de façon à ce que le mélange se forme in-situ au niveau des fibres ait un pH inférieur à 7, comme défini ci-dessus.

Les exemples suivants sont destinés à illustrer l'invention.

### EXEMPLES 1 à 23

On procède à la teinture des cheveux en appliquant sur des cheveux gris à 90% de blancs un mélange extemporané poids pour poids de la composition colorante (A) et de la composition oxydante (B).

Ce mélange présente le pH indiqué dans le tableau des exemples qui suivent. On laisse agir ce mélange pendant 30 minutes, puis on rince les cheveux, on effectue un shampooing. Après séchage, les cheveux sont teints dans la nuance précisée au bas du tableau ci-joint.

| A) **Composition colorante** (composant A) | 1 | 2 | 3 |
|---|---|---|---|
| 6-hydroxy 5-acétoxyindole | 0,573 | | |
| 6-hydroxy-N-méthylindole | | 0,441 | |
| 6-hydroxy-5-méthoxy-2-méthylindole | | | 0,708 |
| Paraphénylènediamine | 0,324 | | |
| 2,6-diméthyl paraphénylènediamine | | 0,657 | |
| 2-méthylparaphénylènediamine2 HCl | | | 0,488 |
| Monoéthanolamine qs pH | 9,7 | 8,9 | 9,2 |
| Milieu de coloration 1 | X | | |
| Milieu de coloration 2 | | X | X |
| Eau qsp | 100 | 100 | 100 |

| B) **Composition oxydante** (composant B) | | | |
|---|---|---|---|
| Solution d'eau oxygénée à 20 volumes | | | |
| Acide phosphorique qs pH | 1 | 1 | 1 |
| **pH mélange p/p A + B** | **4,3** | **6,7** | **6,7** |
| nuances obtenues | blond | blond | chatain |
| | foncé | foncé | cendré |
| | irisé | cuivré | mat |
| | cendré | irisé | |

| A) **Composition colorante** (composant A) | 4 | 5 | 6 |
|---|---|---|---|
| 6-hydroxy 2-carboxy indole | 0,354 | | |
| 6-hydroxy-5-méthoxy-2,3-diméthylindole | | 0,573 | |
| 7-hydroxy-3-méthylindole | | | 0,294 |
| Paraphénylènediamine | 0,216 | | 0,216 |
| 2,6-diméthylparaphénylènediamine | | 0,657 | |
| 3-(β-hydroxyéthylamino)6-méthylphénol | 0,05 | | |
| Métaaminophénol | 0,05 | | |
| α-naphtol | 0,05 | | |
| Monoéthanolamine qs pH | 9 | 8,9 | 9,2 |
| Milieu de coloration 2 | X | X | X |
| Eau qsp | 100 | 100 | 100 |

| B) **Composition oxydante** (composant B) | | | |
|---|---|---|---|
| Solution d'eau oxygénée à 20 volumes | | | |
| Acide phosphorique qs pH | 1 | 1 | 1 |
| **pH mélange p/p A + B** | **6,5** | **6,4** | **6,7** |
| nuances obtenues | chatain | blond | blond |
| | clair | foncé | beige |
| | beige | cendré | |
| | nacré | | |

| A) **Composition colorante** (composant A) | 7 | 8 | 9 |
|---|---|---|---|
| 6-hydroxyindole | 0,532 | | |
| 6-hydroxy-7-méthoxyindole | | 0,489 | |
| 7-hydroxyindole | | | 0,399 |
| Paraaminophénol | 0,432 | | |
| 2,6-diméthylparaphénylènediamine | | 0,657 | |
| Paraphénylènediamine | | | 0,327 |
| Monoéthanolamine qs pH | 9,2 | 9,4 | 9,8 |
| Milieu de coloration 1 | | X | X |
| Milieu de coloration 2 | X | | |
| Eau qsp | 100 | 100 | 100 |

| B) **Composition oxydante** (composant B) | | | |
|---|---|---|---|
| Solution d'eau oxygénée à 20 volumes | | | |
| Acide phosphorique qs pH | 1 | 1,5 | 1,5 |
| **pH mélange p/p A + B** | **6,7** | **6,6** | **5** |
| nuances obtenues | blond | bleu | chatain |
| | très | | |
| | clair | | |
| | doré | | |

| A) **Composition colorante** (composant A) | 10 | 11 |
|---|---|---|
| 6-hydroxyindole | | 0,399 |
| 6-hydroxy 5-methoxy N-méthylindole | 0,531 | |
| 2,6-diméthyl paraphénylènediamine | 0,657 | 0,657 |
| Monoéthanolamine qs pH | 8,9 | 9,8 |
| Milieu de coloration 1 | | X |
| Milieu de coloration 2 | X | |
| Eau qs | 100 | 100 |

| B) **Composition oxydante** (composant B) | | |
|---|---|---|
| solution d'eau oxygénée à 20 volumes | | |
| Acide phosphorique qs pH | 1 | 1,5 |
| **pH mélange p/p A + B** | **6,3** | **6,5** |
| nuances obtenues | blond | rouge |
| | foncé | puissant |
| | acajou | légèrement |
| | irisé | cuivré |

| A) Composition colorante (composant A) | 12 | 13 | 14 | 15 |
|---|---|---|---|---|
| 6-hydroxyindole | | | | |
| 6-hydroxy 7-méthylindole | 0,44 | | | |
| 2-carboxy 5-méthoxy 6-hydroxyindole | | 0,62 | | |
| 5-méthoxy 6-hydroxyindole | | | 0,49 | |
| 5-acétoxy 6-hydroxyindole | | | | 0,57 |
| 2,3-diméthyl 5-acétylamino 6-hydroxyindole | | | | |
| Paraphénylènediamine | 0,32 | 0,32 | 0,32 | |
| 2,6-diméthylparaphénylènediamine, 2HCl | | | | 0,63 |
| 2-méthoxyméthyl paraaminophénol | | | | |
| Monoéthanolamine qs pH | 9,2 | 9,2 | 9,2 | 8,7 |
| Milieu de coloration 3 | X | X | X | X |
| Eau qsp | 100 | 100 | 100 | 100 |

| B) Composition oxydante (composant B) | | | | |
|---|---|---|---|---|
| Solution d'eau oxygénée à 20 volumes | | | | |
| Acide phosphorique qs pH | 1 | 1 | 1 | 1 |
| pH mélange p/p A + B | 6,8 | 6,8 | 6,8 | 6,3 |
| Nuances obtenues sur cheveux permanentés 90% blanc | châtain | châtain | irisé | violine |
| | acajou | clair | rouge | irisé |
| | rouge | irisé | puissant | |
| | | cendré | | |

| A) Composition colorante (composant A) | 16 | 17 | 18 |
|---|---|---|---|
| 6-hydroxyindole | | 0,4 | |
| 6-hydroxy 7-méthylindole | | | |
| 2-carboxy 5-méthoxy 6-hydroxyindole | | | |
| 5-méthoxy 6-hydroxyindole | | | |
| 5-acétoxy 6-hydroxyindole | 0,57 | | |
| 2,3-diméthyl 5-acétylamino 6-hydroxyindole | | | 0,65 |
| Paraphénylènediamine | | | |
| 2,6-diméthylparaphénylènediamine, 2HCl | | | 0,63 |
| 2-méthoxyméthyl paraaminophénol | 0,46 | 0,46 | |
| Monoéthanolamine qs pH | 9,3 | 9,3 | 9,1 |
| Milieu de coloration 3 | X | X | X |
| Eau qsp | 100 | 100 | 100 |

| B) Composition oxydante (composant B) | | | |
|---|---|---|---|
| Solution d'eau oxygénée à 20 volumes | | | |
| Acide phosphorique qs pH | 1 | 1,5 | 1 |
| pH mélange p/p A + B | 6,5 | 6,9 | 6,6 |
| Nuances obtenues sur cheveux permanentés 90% blanc | blond | blond | châtain |
| | clair | très clair | clair |
| | beige | naturel | cendré |
| | cendré | cendré | |
| | | doré | |

| A) Composition colorante (composant A) | 19 | 20 | 21 |
|---|---|---|---|
| 5-hydroxyindole | 0,399 | | |
| 3-méthyl 5-hydroxyindole | | 0,44 | |
| 2-éthoxycarbonyl 5-hydroxyindole | | | 0,615 |
| 2,3-diméthyl 5-hydroxy 6-méthoxyindole | | | |
| 5-hydroxy 6-méthoxyindole | | | |
| Paraphénylènediamine | 0,324 | | |
| 2,6-diméthylparaphénylènediamine, 2HCl | | | 0,627 |
| Paraaminophénol | | 0,327 | |
| Monoéthanolamine qs pH | 9,2 | 9,2 | 9,1 |
| Milieu de coloration 3 | X | X | X |
| Eau qsp | 100 | 100 | 100 |

| B) Composition oxydante (composant B) | | | |
|---|---|---|---|
| Solution d'eau oxygénée à 20 volumes | | | |
| Acide phosphorique qs pH | 1 | 1 | 1 |
| pH mélange p/p A + B | 6,6 | 6,6 | 6,5 |
| Nuances obtenues sur cheveux naturels 90% blanc | | beige | cendré |
| | violine | nacré | violine |
| | | | léger |

| A) Composition colorante (composant A) | 22 | 23 |
|---|---|---|
| 5-hydroxyindole | | |
| 3-méthyl 5-hydroxyindole | | |
| 2-éthoxycarbonyl 5-hydroxyindole | | |
| 2,3-diméthyl 5-hydroxy 6-méthoxy indole | | 0,573 |
| 5-hydroxy 6-méthoxyindole | 0,489 | |
| Paraphénylènediamine | | |
| 2,6-diméthylparaphénylènediamine, 2HCl | 0,627 | 0,627 |
| Paraaminophénol | | |
| Monoéthanolamine qs pH | 9 | 9 |
| Milieu de coloration 3 | X | X |
| Eau qsp | 100 | 100 |

| B) Composition oxydante (composant B) | | |
|---|---|---|
| Solution d'eau oxygénée à 20 volumes | | |
| Acide phosphorique qs pH | 1 | 1 |
| pH mélange p/p A + B | 6,7 | 6,6 |
| Nuances obtenues sur cheveux naturels 90% blanc | bleu | gris |
| | | mat |
| | | cendré |

| MILIEU DE COLORATION N^{o} 1 | |
|---|---|
| - Nonylphénol à 4 moles d'oxyde d'éthylène, vendu sous la dénomination SINNOPAL NP4 par la Société HENKEL | 25,5 g |
| - Nonylphénol à 9 moles d'oxyde d'éthylène, vendu sous la dénomination SINNOPAL NP9 par la Société HENKEL | 17,5 g |
| - Butyléther d'éthylèneglycol | 7,0 g |
| - Propylèneglycol | 11,0 g |
| - Alcool éthylique | 2,0 g |
| - Lauryléthersulfate de monoéthanolamine vendu sous la dénomination SACTIPON 2 OM 29 par la Société LEVER à 28% de MA | 5,0 g MA |
| - Métabisulfite de sodium en solution aqueuse à 35% de MA | 0,46 g MA |
| - Acétate de sodium | 0,8 g |
| - Antioxydant, séquestrant, qs | |

| MILIEU DE COLORATION N^{o} 2 | |
|---|---|
| - Alcool oléique polyglycérolé à 2 moles de glycérol | 4,0 g |
| - Alcool oléique polyglycérolé à 4 moles de glycérol, à 78% de MA | 5,69 g MA |
| - Acide oléique | 3,0 g |
| - Amine oléique 2 OE vendue sous la dénomination ETHOMEEN O 12 par la Société AKZO | 7,0 g |
| - Laurylaminosuccinamate de diéthylaminopropyle, sel de sodium à 55% de MA | 3,0 g MA |
| - Alcool oléique | 5,0 g |
| - Diéthanolamide d'acide de coprah | 12,0 g |
| - Propylèneglycol | 4,0 g |
| - Alcool éthylique | 7,0 g |
| - Monométhyléther de propylèneglycol | 9,0 g |
| - Bisulfite de sodium en solution aqueuse à 35% de MA | 0,46 g MA |
| - Acétate d'ammonium | 0,8 g |
| - Antioxydant, séquestrant, qs | |

| MILIEU DE COLORATION N^{o} 3 | |
|---|---|
| - Alcool oléique polyglycérolé à 2 moles de glycérol | 4,0 g |
| - Alcool oléique polyglycérolé à 4 moles de glycérol à 78% de MA | 5,69 g MA |
| - Acide oléique | 3,0 g |
| - Amine oléique 2 OE vendue sous la dénomination ETHOMEEN O 12 par la Société AKZO | 7,0 g |
| - Laurylaminosuccinamate de diéthylaminopropyle, sel de sodium à 55% de MA | 3,0 g MA |
| - Alcool oléique | 5,0 g |
| - Diéthanolamide d'acide oléique | 12,0 g |
| - Propylèneglycol | 3,5 g |
| - Alcool éthylique | 7,0 g |
| - Dipropylèneglycol | 0,5 g |
| - Monométhyléther de propylèneglycol | 9,0 g |
| - Métabisulfite de sodium en solution aqueuse à 35% de MA | 0,46 g MA |
| - Acétate d'ammonium | 0,8 g |
| - Antioxydant, séquestrant, qs | |

## Revendications

1. Procédé de teinture des fibres kératiniques, en particulier des fibres kératiniques humaines telles que les cheveux, caractérisé par le fait qu'on applique sur ces fibres une composition contenant dans un milieu approprié pour la teinture, au moins un couleur indolique, répondant à la formule : 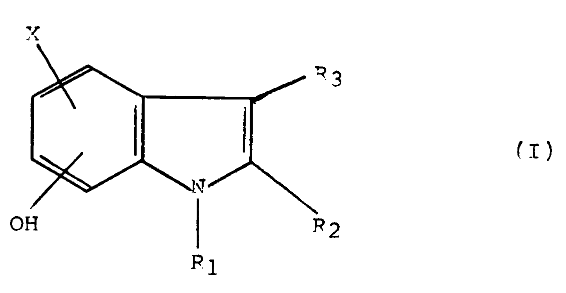 dans laquelle R₁ désigne un atome d'hydrogène, un radical alkyle en C₁-C₄; R₂ et R₃, identiques ou différents, désignent un atome d'hydrogène, un radical alkyle inférieur en C₁-C₄, un radical carboxyle ou un radical alcoxycarbonyle; X désigne un atome d'hydrogène, un radical alkyle inférieur en C₁-C₄, un radical alcoxy en C₁-C₁₈, un atome d'halogène, un radical acyloxy en C₂-C₂₀, un groupement acétylamino; le groupement OH occupant les positions 5 ou 6 ou 7 du cycle aromatique; ainsi que les sels de ces composés;
un précurseur de colorant d'oxydation;
un agent oxydant;
le pH de la composition appliqué sur les fibres étant inférieur à 7.

2. Procédé selon la revendication 1, caractérisé par le fait que les composés de formule (I) sont choisis parmi les composés dans lesquels le radical alkyle désigne méthyle, éthyle; le radical alcoxy carbonyle désigne méthoxy ou éthoxycarbonyle; le radical alcoxy désigne méthoxy, éthoxy, butoxy, hexadécyloxy; le radical acyloxy désigne acétoxy, tétradécanoyloxy.

3. Procédé selon la revendication 2, caractérisé par le fait que les composés de formule (I) sont choisis parmi le 6-hydroxyindole, le 6-hydroxy 3-méthoxycarbonylindole, le 6-hydroxy 1-méthyl 3-méthoxycarbonylindole, le 6-hydroxy 1-méthyl 2,3-diméthoxycarbonylindole, le 6-hydroxy 1,2-diméthyl indole, le 6-hydroxy 2-méthylindole, le 6-hydroxy 2-carboxyindole, le 6-hydroxy 2,3-diméthylindole, le 6-hydroxy 3-carboxyindole, le 6-hydroxy 3-éthoxy carbonylindole, le 6-hydroxy 2-éthoxycarbonylindole, le 6-hydroxy 3-méthylindole, le 6-hydroxy 1-méthylindole, le 6-hydroxy 5-acétoxyindole, le 6-hydroxy 5-méthoxy indole, le 6-hydroxy 5-méthoxy 2-méthylindole, le 6-hydroxy 5-butoxyindole, l'acide 6-hydroxy 5-méthoxy indole 2-carboxylique, le 6-hydroxy 5-méthoxy 2,3-diméthylindole, le 6-hydroxy 7-méthoxyindole, le 6-hydroxy 5-tétradécanoyloxyindole, le 6-hydroxy 5-hexadécyloxyindole, le 7-hydroxyindole, le 7-hydroxy 3-méthylindole, le 7-hydroxy 4-méthoxy 2,3-diméthyl indole, le 6-hydroxy 5-méthoxy 1-méthylindole, le 6-hydroxy 7-méthylindole, le 6-hydroxy 2-éthoxycarbonyl 5-méthoxyindole, le 6-hydroxy 2-méthoxycarbonyl 5-butylindole, le 6-hydroxy 2,3-diméthyl 5-(acétylamino) indole, le 5-hydroxy 2-méthyl 3-éthoxycarboxylindole, le 5-hydroxy 2-carboxyindole.

4. Procédé selon l'une quelconque des revendications 1 à 3, caractérisé par le fait que les précurseurs de colorants d'oxydation sont choisis parmi les paraphénylènediamines, les para-aminophénols, les précurseurs hétérocycliques para.

5. Procédé selon la revendication 4, caractérisé par le fait que les paraphénylènediamines sont choisies parmi les composés répondant à la formule: 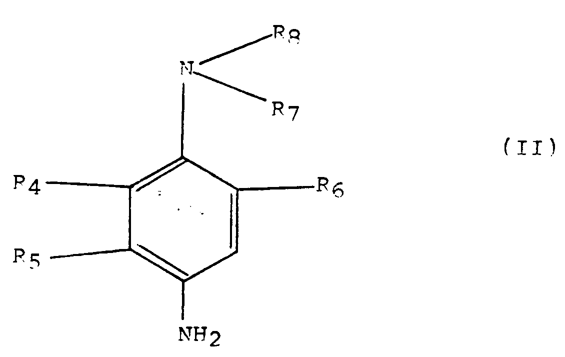 dans laquelle R₄, R₅ et R₆, identiques ou différents, représentent un atome d'hydrogène ou d'halogène, un radical alkyle ayant de 1 à 4 atomes de carbone, un radical alcoxy ayant de 1 à 4 atomes de carbone; R₇ et R₈, identiques ou différents, représentent un atome d'hydrogène, un radical alkyle, hydroxyalkyle, alcoxy alkyle, carbamylalkyle, mésylaminalkyle, acétylamino alkyle, uréidoalkyle, carbalcoxyaminoalkyle, pipéridino alkyle, morpholinoalkyle; ces groupes alkyle ou alkoxy ayant de 1 à 4 atomes de carbone, ou bien R₇ et R₈ forment, conjointement avec l'atome d'azote auquel ils sont liés, un hétérocycle pipéridino ou morpholino, sous réserve que R₄ ou R₆ représente un atome d'hydrogène lorsque R₇ et R₈ ne représentent pas un atome d'hydrogène, ainsi que les sels de ces composés.

6. Procédé selon la revendication 4 ou 5, caractérisé par le fait que les composés de formule (II) sont choisis parmi la p-phénylènediamine, la p-toluylènediamine, la méthoxyparaphénylènediamine, la chloroparaphénylènediamine, la 2,6-diméthylparaphénylènediamine, la 2,5-diméthylparaphénylènediamine, la 2,3-diméthylparaphénylènediamine, la 2-méthyl 5-méthoxyparaphénylènediamine, la 2,6-diméthyl 5-méthoxyparaphénylènediamine, la N,N-diméthyl paraphénylènediamine, la 3-méthyl 4-amino N,N-diéthyl aniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4′-amino)phényl] morpholine, la N-[(4′-amino)phényl] pipéridine, sous forme de base libre ou de sels.

7. Procédé selon la revendication 4 ou 5, caractérisé par le fait que les p-aminophénols sont choisis parmi le p-aminophénol, le 2-méthyl 4-amino phénol, le 3-méthyl 4-aminophénol, le 2-chloro 4-amino phénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)4-aminophénol, le 2-éthoxy 4-aminophénol, le 3-méthoxy 4-aminophénol, le 2,5-diméthyl 4-aminophénol, le 2-méthoxyméthyl 4-aminophénol.

8. Procédé selon l'une quelconque des revendications 1 à 3, caractérisé par le fait que les précurseurs de colorants d'oxydation sont des précurseurs de colorants d'oxydation de type ortho choisis parmi les ortho-aminophérols et les ortho-phénylènediamines.

9. Procédé selon l'une quelconque des revendications 1 à 8, caractérisé par le fait que l'agent oxydant est choisi parmi le peroxyde d'hydrogène, le peroxyde d'urée, les bromates de métaux alcalins, les persels.

10. Procédé selon l'une quelconque des revendications 1 à 9, caractérisé par le fait que le pH de la composition appliqué sur les fibres kératiniques est compris entre 3 et 6,9.

11. Procédé selon l'une quelconque des revendications 1 à 10, caractérisé par le fait que la composition utilisée pour la teinture des fibres kératiniques contient en plus des coupleurs hétérocycliques de la famille des 6- ou 7-hydroxy indoles de formule (I), d'autres coupleurs choisis parmi les métadiphénols, les métaaminophénols, les métaphénylènediamines, les métaaeylaminophénols, les métauréidophénols, les métacarbalcoxyaminophénols, l' α-naphtol, les coupleurs possédant un groupement méthylène actif choisis parmi les composés β-cétoniques et les pyrazolones.

12. Procédé selon la revendication 11, caractérisé par le fait que les coupleurs sont choisis parmi le 2,4-dihydroxyphénoxyéthanol, le 2,4-dihydroxyanisole, le métaaminophénol, la résorcine, le monométhyléther de résorcine, la 2-méthylrésorcine, le 2-méthyl 5-N-(β-hydroxyéthyl)aminophénol, le 2-méthyl 5-N-(β-mésylaminoéthyl)aminophénol, la 6-hydroxylbenzomorpholine, le 2,4-diaminoanisole, le 2,4-diaminophénoxyéthanol, la 6-aminobenzomorpholine, le [2-N-(β-hydroxyéthyl)amino 4-amino]-phénoxyéthanol, le 2-amino 4-N-(β-hydroxyéthyl)aminoanisole, le (2,4-diamino)pnényl-β, γ-dihydroxypropyléther, la 2,4-diaminophénoxyéthylamine, le 2-méthyl 5-aminophénol, le 2,6-diméthyl 3-aminophénol, le 3,4-méthylènedioxyphénol, le 3,4-méthylènedioxyaniline et leurs sels.

13. Procédé selon l'une quelconque des revendications 1 à 12, caractérisé par le fait que la composition contient des agents tensio-actifs anioniques, cationiques, nom ioniques, amphotères ou leurs mélanges; des agents épaississants, des agents anti-oxydants et/ou tout autre adjuvant cosmétiquement acceptable.

14. Procédé selon l'une quelconque des revendications 1 à 13, caractérisé par le fait que le milieu approprié pour la teinture est constitué par de l'eau ou un mélange d'eau et d'un solvant choisi parmi les alcanols inférieurs en C₂-C₄, le glycérol, les glycols ou éthers de glycol, le monoéthyléther et le monométhyléther de diéthylèneglycol, les alcools aromatiques ou le phénoxyéthanol, ou leurs mélanges.

15. Agent de teinture des fibres kératiniques et en particulier des cheveux, caractérisé par le fait qu'il conporte au moins deux composants; un composant (A) constitué par une composition contenant dans un milieu approprié pour la teinture, un coupleur indolique répondant à la formule (I) tel que défini dans l'une quelconque des revendications 1 à 3 et un précurseur de colorant d'oxydation tel que défini dans l'une quelconque des revendications 1 et 4 à 12, un composant (B) constitué par une composition contenant dans un milieu approprié pour la teinture, un agent oxydant, le pH des composants (A) et (B) étant tel qu'après mélange dans des proportions de 90 à 10% pour le composant (A), de 10 à 90% pour le composant (B), la composition résultante ait un pH inférieur à 7.

16. Agent selon la revendication 15, caractérisé par le fait que le composant (A) a un pH compris entre 3 et 10,5.

17. Agent selon la revendication 15 ou 16, caractérisé par le fait que le composant (A) contient les précurseurs de colorants par oxydation du type para et/ou ortho ainsi que les coupleurs, dans des proportions comprises entre 0,3 et 7% en poids par rapport au poids total du composant (A).

18. Agent selon l'une quelconque des revendications 15 à 17, caractérisé par le fait que la concentration en composés de formule (I) est comprise entre 0,05 et 4% en poids par rapport au poids total du composant A.

19. Agent selon l'une quelconque des revendications 15 à 18, caractérisé par le fait que le composant (A) contient des agents tensio-actifs dans des proportions de 0,1 à 55% en poids, des agents solvants en plus de l'eau dans des proportions comprises entre 0,5 et 40% en poids, des agents épaississants dans des proportions comprises entre 0,1 et 5% en poids, des agents antioxydants dans des proportions comprises entre 0,02 et 1,5% en poids, et/ou tout autre adjuvant cosmétiquement acceptable.

20. Agent selon l'une quelconque des revendications 15 à 19, caractérisé par le fait que le composant (B) a un pH qui a une valeur minimum de 1 et inférieure à 7.

21. Procédé de teinture des fibres kératiniques et en particulier des cheveux, caractérisé par le fait qu'il comporte une première étape consistant à stocker un agent de teinture tel que défini dans l'une quelconque des revendications 15 à 20 et à procéder avant application au mélange des composants (A) et (B) dans des proportions de 10 à 90% pour le composant (A) et de 90 à 10% pour le composant (B), de façon à obtenir une composition ayant un pH inférieur à 7 et d'appliquer ce mélange immédiatement après préparation sur les fibres kératiniques.

22. Dispositif à plusieurs compartiments ou kit de teinture, caractérisé par le fait qu'il comporte au moins deux compartiments dont un premier compartiment renferme le composant (A) tel que défini dans l'une quelconque des revendications 15 à 20, et le second compartiment renferme le composant (B) tel que défini dans les revendications 5 à 20.

23. Dispositif selon la revendication 22, caractérisé par le fait qu'il est équipé d'un moyen permettant de délivrer sur les cheveux le mélange souhaité des composants (A) et (B).

24. Procédé de teinture selon l'une quelconque des revendications 1 à 14, caractérisé par le fait que l'on applique sur les cheveux la composition et qu'on la laisse poser pendant 3 à 40 minutes, que l'on rince les cheveux et qu'on procède éventuellement à un shampooing avant un nouveau rinçage et séchage.

25. Utilisation comme coupleurs de dérivés de 5-, 6- ou 7-hydroxyindoles répondant à la formule (I) tels que définis dans l'une quelconque des revendications 1 à 3 pour la teinture en milieu acide des fibres kératiniques, en association avec des précurseurs de colorants d'oxydation.

26. Composition de teinture de fibres kératiniques, prête à l'emploi telle que mise en oeuvre dans le procédé selon l'une quelconque des revendications 1 à 14.

27. Composition selon la revendication 26, contenant le composé de formule I dans des proportions de 0,01 à 3,5% en poids par rapport au poids total de la composition.

## Claims

1. Process for dyeing keratinous fibres, in particular human keratinous fibres such as hair, characterised in that there is applied to these fibres a composition containing, in a suitable dyeing medium, at least one indole-based coupler, of the formula: 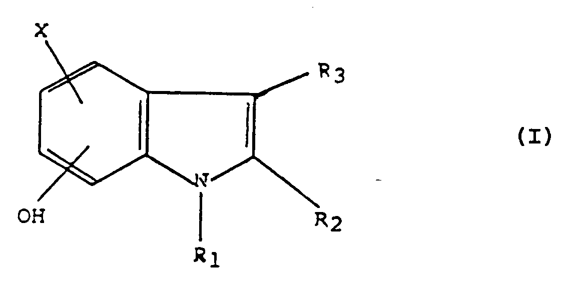 in which R₁ designates a hydrogen atom or a C₁-C₄ alkyl radical; R₂ and R₃, which may be identical or different, designate a hydrogen atom, a C₁-C₄ lower alkyl radical, a carboxyl radical or an alkoxycarbonyl radical; X designates a hydrogen atom, a C₁-C₄ lower alkyl radical, a C₁-C₁₈ alkoxy radical, a halogen atom, a C₂-C₂₀ acyloxy radical or an acetylamino group; the OH group occupying the 5- or 6- or 7- positions of the aromatic ring; as well as the salts of these compounds;
an oxidation dye precursor;
an oxidising agent;
the pH of the composition applied to the fibres being less than 7.

2. Process according to Claim 1, characterised in that the compounds of formula (I) are chosen from among the compounds in which the alkyl radical designates methyl or ethyl; the alkoxycarbonyl radical designates methoxy or ethoxycarbonyl; the alkoxy radical designates methoxy, ethoxy, butoxy or hexadecyloxy; the acyloxy radical designates acetoxy or tetradecanoyloxy.

3. Process according to Claim 2, characterised in that the compounds of formula (I) are chosen from among 6-hydroxyindole, 6-hydroxy-3-methoxycarbonylindole, 6-hydroxy-1-methyl-3-methoxycarbonylindole, 6-hydroxy-1-methyl-2,3-dimethoxycarbonylindole, 6-hydroxy-1,2-dimethylindole, 6-hydroxy-2-methylindole, 6-hydroxy-2-carboxyindole, 6-hydroxy-2,3-dimethylindole, 6-hydroxy-3-carboxyindole, 6-hydroxy-3-ethoxycarbonylindole, 6-hydroxy-2-ethoxycarbonylindole. 6-hydroxy-3-methylindole, 6-hydroxy-1-methylindole, 6-hydroxy-5-acetoxyindole, 6-hydroxy-5-methoxyindole, 6-hydroxy-5-methoxy-2-methylindole, 6-hydroxy-5-butoxyindole, 6-hydroxy-5-methoxyindole-2-carboxylic acid, 6-hydroxy-5-methoxy-2,3-dimethylindole, 6-hydroxy-7-methoxyindole, 6-hydroxy-5-tetradecanoyloxyindole, 6-hydroxy-5-hexadecyloxyindole, 7-hydroxyindole, 7-hydroxy-3-methylindole, 7-hydroxy-4-methoxy-2,3-dimethylindole, 6-hydroxy-5-methoxy-1-methylindole, 6-hydroxy-7-methylindole, 6-hydroxy-2-ethoxycarbonyl-5-methoxyindole, 6-hydroxy-2-methoxycarbonyl-5-butylindole, 6-hydroxy-2,3-dimethyl-5-(acetylamino)indole, 5-hydroxy-2-methyl-3-ethoxycarbonylindole and 5-hydroxy-2-carboxyindole.

4. Process according to any one of Claims 1 to 3, characterised in that the oxidation dye precursors are chosen from among para-phenylenediamines, para-aminophenols and para-heterocyclic precursors.

5. Process according to Claim 4, characterised in that the para-phenylenediamines are chosen from among the compounds of the formula: 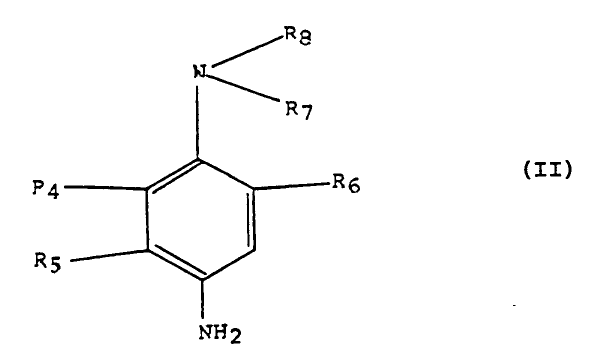 in which R₄, R₅ and R₆, which may be identical or different, represent a hydrogen or halogen atom, an alkyl radical having from 1 to 4 carbon atoms or an alkoxy radical having from 1 to 4 carbon atoms; R₇ and R₈, which may be identical or different, represent a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl or morpholinoalkyl radical; these alkyl or alkoxy groups having from 1 to 4 carbon atoms, or R₇ and R₈, form, together with the nitrogen atom to which they are attached, a piperidino or morpholino heterocycle, on the condition that R₄ or R₆ represents a hydrogen atom when R₇ and R₈ do not represent a hydrogen atom, as well as the salts of these compounds.

6. Process according to Claim 4 or 5, characterised in that the compounds of formula (II) are chosen from among p-phenylenediamine, p-toluylenediamine, methoxy-para-phenylenediamine, chloro-para-phenylenediamine, 2,6-dimethyl-para-phenylenediamine, 2,5-dimethyl-para-phenylenediamine, 2,3-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline, N,N-di-(β-hydroxyethyl)-para-phenylenediamine, 3-methyl-4-amino-N,N-di-(β-hydroxyethyl)aniline, 3-chloro-4-amino-N,N-di-(β-hydroxyethyl)aniline, 4-amino-N-ethyl-N-carbamylmethylaniline, 3-methyl-4-amino-N-ethyl-N-carbamylmethylaniline, 4-amino-N-ethyl-N-β-piperidinoethylaniline, 3-methyl-4-amino-N-ethyl-N-β-piperidinoethylaniline, 4-amino-N-ethyl-N-β-morpholinoethylaniline, 3-methyl-4-amino-N-ethyl-N-β-morpholinoethylaniline, 4-amino-N-ethyl-N-β-acetylaminoethylaniline, 4-amino-N-(β-methoxy-ethyl)aniline, 3-methyl-4-amino-N-ethyl-N-β-acetylaminoethylaniline, 4-amino-N-ethyl-N-β-mesylaminoethylaniline, 3-methyl-4-amino-N-ethyl-N-β-mesylaminoethylaniline, 4-amino-N-ethyl-N-β-sulphoethylaniline, 3-methyl-4-amino-N-ethyl-N-β-sulphoethylaniline, N-[(4'-amino)phenyl]morpholine and N-[(4'-amino)phenyl]piperidine, in the form of a free base or salts.

7. Process according to Claim 4 or 5, characterised in that the p-aminophenols are chosen from among p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol and 2-methoxymethyl-4-aminophenol.

8. Process according to any one of Claims 1 to 3, characterised in that the oxidation dye precursors are ortho type oxidation dye precursors chosen from among ortho-aminophenols and ortho-phenylenediamines.

9. Process according to any one of Claims 1 to 8, characterised in that the oxidising agent is chosen from among hydrogen peroxide, urea peroxide, alkaline metal bromates and per salts.

10. Process according to any one of Claims 1 to 9, characterised in that the pH of the composition applied to the keratinous fibres is between 3 and 6.9.

11. Process according to any one of Claims 1 to 10, characterised in that the composition used for dyeing keratinous fibres contains, in addition to the heterocyclic couplers of the 6- or 7-hydroxyindole family of formula (I), other couplers chosen from among meta-diphenols, meta-aminophenols, meta-phenylenediamines, meta-acylaminophenols, meta-ureidophenols, meta-carbalkoxyaminophenols or α-naphthol or couplers containing an active methylene group chosen from among β-ketonic compounds and pyrazolones.

12. Process according to Claim 11, characterised in that the couplers are chosen from among 2,4-dihydroxyphenoxyethanol, 2,4-dihydroxyanisole, meta-aminophenol, resorcinol, resorcinol monomethyl ether, 2-methyl-resorcinol, 2-methyl-5-N-(β-hydroxyethyl)aminophenol, 2-methyl-5-N-(β-mesylaminoethyl)aminophenol, 6-hydroxy-benzomorpholine, 2,4-diaminoanisole, 2,4-diaminophenoxyethanol, 6-aminobenzomorpholine, [2-N-(β-hydroxyethyl)amino-4-amino]phenoxyethanol, 2-amino-4-N-(β-hydroxyethyl)aminoanisole, (2,4-diamino)phenyl-β,γ-dihydroxypropyl ether, 2,4-diaminophenoxyethylamine, 2-methyl-5-aminophenol, 2,6-dimethyl-3-aminophenol, 3,4-methylene-dioxyphenol and 3,4-methylenedioxyaniline and their salts.

13. Process according to any one of Claims 1 to 12, characterised in that the composition contains anionic, cationic, non-ionic or amphoteric surface-active agents or their mixtures; thickening agents, antioxidants and/or any other cosmetically acceptable adjuvant.

14. Process according to any one of Claims 1 to 13, characterised in that the suitable dyeing medium consists of water or a mixture of water and a solvent chosen from among C₂-C₄ lower alkanols, glycerol, glycols or glycol ethers, diethylene glycol monoethyl ether and monomethyl ether, aromatic alcohols or phenoxy ethanol, or their mixtures.

15. Agent for dyeing keratinous fibres and in particular hair, characterised in that it comprises at least two components; one component (A) which consists of a composition containing, in a suitable dyeing medium, an indole-based coupler of the formula (I) as defined in any one of Claims 1 to 3 and an oxidation dye precursor as defined in any one of Claims 1 and 4 to 12, a component (B) which consists of a composition containing, in a suitable dyeing medium, an oxidising agent, the pH of the components (A) and (B) being such that after mixing in proportions of 90 to 10% for the component (A) and of 10 to 90% for the component (B), the resulting composition has a pH of less than 7.

16. Agent according to Claim 15, characterised in that the component (A) has a pH of between 3 and 10.5.

17. Agent according to Claim 15 or 16, characterised in that the component (A) contains the para and/or ortho type oxidation dye precursors as well as the couplers in proportions of between 0.3 and 7% by weight relative to the total weight of the component (A).

18. Agent according to any one of Claims 15 to 17, characterised in that the concentration of compounds of formula (I) is between 0.05 and 4% by weight relative to the total weight of the component A.

19. Agent according to any one of Claims 15 to 18, characterised in that the component (A) contains surface-active agents in proportions of 0.1 to 55% by weight, solvents in addition to water in proportions of between 0.5 and 40% by weight, thickening agents in proportions of between 0.1 and 5% by weight, antioxidants in proportions of between 0.02 and 1.5% by weight, and/or any other cosmetically acceptable adjuvant.

20. Agent according to any one of Claims 15 to 19, characterised in that the component (B) has a pH which has a minimum value of 1 and is less than 7.

21. Process for dyeing keratinous fibres and in particular hair, characterised in that it comprises a first stage consisting in storing a dyeing agent as defined in any one of Claims 15 to 20 and in carrying out, before application, the mixing of the components (A) and (B) in proportions of 10 to 90% for the component (A) and of 90 to 10% for the component (B), so as to obtain a composition which has a pH of less than 7 and applying this mixture to the keratinous fibres immediately after preparation.

22. Multi-compartment device or dyeing kit, characterised in that it comprises at least two compartments of which a first compartment contains the component (A) as defined in any one of Claims 15 to 20, and the second compartment contains the component (B) as defined in Claims 5 to 20.

23. Device according to Claim 22, characterised in that it is equipped with a means permitting the desired mixture of components (A) and (B) to be delivered to the hair.

24. Dyeing process according to any one of Claims 1 to 14, characterised in that the composition is applied to the hair and allowed to settle for 3 to 40 minutes, the hair is rinsed and a shampooing is optionally carried out before rinsing again and drying.

25. Use, as couplers, of 5-, 6- or 7-hydroxyindole derivatives of the formula (I) as defined in any one of Claims 1 to 3 for the dyeing in acid medium of keratinous fibres, in combination with oxidation dye precursors.

26. Ready-for-use composition for dyeing keratinous fibres, as employed in the process according to any one of Claims 1 to 14.

27. Composition according to Claim 26, containing the compound of formula I in proportions of 0.01 to 3.5% by weight relative to the total weight of the composition.

## Patentansprüche

1. Verfahren zur Färbung keratinischer Fasern, insbesondere menschlicher keratinischer Fasern wie der Haare,
dadurch **gekennzeichnet**, daß
man auf die Haare eine Zusammensetzung aufträgt, die in einem zur Färbung geeigneten Milieu mindestens einen indolischen Kuppler der Formel: 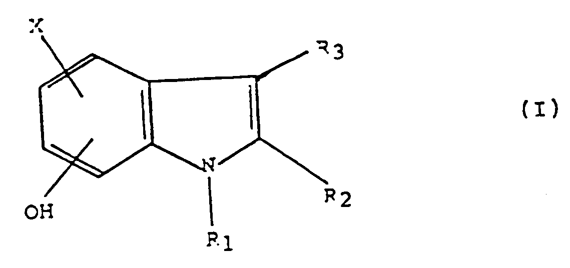 worin R₁ ein Wasserstoffatom, einen C₁₋₄-Alkylrest, R₂ und R₃, gleich oder verschieden, ein Wasserstoffatom, einen C₁₋₄-Niedrigalkyl-, Carboxyl- oder Alkoxycarbonylrest, X ein Wasserstoffatom, einen C₁₋₄-Niedrigalkylrest, einen C₁₋₁₈-Alkoxyrest, ein Halogenatom, einen C₂₋₂₀-Acyloxyrest, eine Acetylaminogruppe darstellen, wobei die OH-Gruppe die Positionen 5 oder 6 oder 7 des aromatischen Rings besetzt, sowie die Salze dieser Verbindungen,
eine Oxidationsfarbstoff-Vorstufe und
ein Oxidationsmittel enthält,
wobei der pH-Wert der auf die Fasern aufgetragenen Zusammensetzung unterhalb 7 liegt.

2. Verfahren gemäß Anspruch 1,
dadurch **gekennzeichnet**, daß
die Verbindungen der Formel (I) aus Verbindungen ausgewählt sind, in de nen der Alkylrest eine Methyl- oder Ethylgruppe, der Alkoxycarbonylrest eine Methoxy- oder Ethoxycarbonylgruppe, der Alkoxyrest eine Methoxy-, Ethoxy-, Butoxy- oder Hexadecyloxygruppe und der Acyloxyrest eine Acetoxy- oder Tetradecanoyloxygruppe darstellen.

3. Verfahren gemäß Anspruch 2,
dadurch **gekennzeichnet**, daß
die Verbindungen der Formel (I) aus 6-Hydroxyindol, 6-Hydroxy-3-methoxycarbonylindol, 6-Hydroxy-1-methyl-2,3-dimethoxycarbonylindol, 6-Hydroxy-1,2-dimethylindol, 6-Hydroxy-2-methylindol, 6-Hydroxy-2-carboxyindol, 6-Hydroxy-2,3-dimethylindol, 6-Hydroxy-3-carboxyindol; 6-Hydroxy-3-ethoxycarbonylindol, 6-Hydroxy-2-ethoxycarbonylindol, 6-Hydroxy-3-methylindol, 6-Hydroxy-1-methylindol, 6-Hydroxy-5-acetoxyindol, 6-Hydroxy-5-methoxyindol, 6-Hydroxy-5-methoxy-2-methylindol, 6-Hydroxy-5-butoxyindol, 6-Hydroxy-5-methoxyindol-2-carboxylsäure, 6-Hyroxy-5-methoxy-2,3-dimethylindol, 6-Hydroxy-7-methoxyindol, 6-Hydroxy-5-tetradecanoyloxyindol, 6-Hydroxy-5-hexadecyloxyindol, 7-Hydroxyindol, 7-Hydroxy-3-methylindol, 7-Hydroxy-4-methoxy-2,3-dimethylindol, 6-Hydroxy-5-methoxy-1-methylindol, 6-Hydroxy-7-methylindol, 6-Hydroxy-2-ethoxycarbonyl-5-methoxyindol, 6-Hydroxy-2-methoxycarbonyl-5-butylindol, 6-Hydroxy-2,3-dimethyl-5-(acetylamino)indol, 5-Hydroxy-2-methyl-3-ethoxycarbonylindol, 5-Hydroxy-2-carboxyindol ausgewählt sind.

4. Verfahren gemäß jedem der Ansprüche 1 bis 3,
dadurch **gekennzeichnet**, daß
die Oxidationsfarbstoff-Vorstufen aus p-Phenylendiaminen, p-Aminophenolen, heterozyklischen Vorstufen vom para-Typ ausgewählt sind.

5. Verfahren gemäß Anspruch 4,
dadurch **gekennzeichnet**, daß
p-Phenylendiamine aus Verbindungen der Formel: 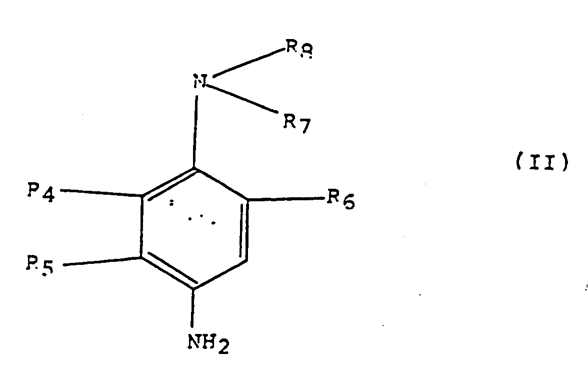 worin R₄, R₅ und R₆, gleich oder verschieden, ein Wasserstoff- oder Halogenatom, einen Alkylrest mit 1 bis 4 Kohlenstoffatomen, einen Alkoxyrest mit 1 bis 4 Kohlenstoffatomen, R₇ und R₈, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyalkylamido-, Piperidinoalkyl-, Morpholinoalkylrest, wobei diese Alkyl- oder Alkoxygruppen 1 bis 4 Kohlenstoffatome aufweisen, darstellen, oder R₇ und R₈ zusammen mit dem Stickstoffatom, an das sie gebunden sind, einen Piperidin- oder Morpholin-Heterozyklus auch bilden, mit der Maßgabe, daß R₄ oder R₆ ein Wasserstoffatom darstellen, wenn R₇ und R₈ kein Wasserstoffatom darstellen, sowie aus den Salzen dieser Verbindungen ausgewählt sind.

6. Verfahren gemäß Anspruch 4 oder 5,
dadurch **gekennzeichnet**, daß
die Verbindungen der Formel (II) aus p-Phenylendiamin, , p-Toluylendiamin, Methoxy-p-phenylendiamin, Chlor-p-phenylendiamin, 2,6-Dimethyl-p-phenylendiamin, 2,5-Dimethyl-p-phenylendiamin, 2,3-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di-(β-hydroxyethyl)-p-phenylendiamin, 3-Methyl-4-amino-N,N-di-(β-hydroxyethyl)anilin, 3-Chlor-4-amino-N,N-di-(β-hydroxyethyl)anilin, 4-Amino-N,N-(ethyl,carbamylmethyl)anilin, 3-Methyl-4-amino-N,N-(ethyl,carbamylmethyl)anilin, 4-Amino-N,N-(ethyl,β-piperidinoethyl)anilin, 3-Methyl-4-amino-N,N-(ethyl,β-piperidinoethyl)anilin, 4-Amino-N,N-(ethyl,β-morpholinoethyl)anilin, 3-Methyl-4-amino-N,N-(ethyl, β-morpholinoethyl)anilin, 4-Amino-N,N-(ethyl,β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)phenyl)morpholin, N-((4'-Amino)phenyl)piperidin, in Form der freien Base oder der Salze, ausgewählt sind.

7. Verfahren gemäß Anspruch 4 oder 5,
dadurch **gekennzeichnet**, daß
die p-Aminophenole aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol ausgewählt sind.

8. Verfahren gemäß jedem der Ansprüche 1 bis 3,
dadurch **gekennzeichne**t, daß
die Oxidationsfarbstoff-Vorstufen Oxidationsfarbstoff-Vorstufen vom ortho-Typ sind, ausgewählt aus o-Aminophenolen und o-Phenylendiaminen.

9. Verfahren gemäß jedem der Ansprüche 1 bis 8,
dadurch **gekennzeichnet**, daß
das Oxidationsmittel aus Wasserstoffperoxid, Harnstoffperoxid, Alkalimetallbromaten, Persalzen ausgewählt ist.

10. Verfahren gemäß jedem der Ansprüche 1 bis 9,
dadurch **gekennzeichnet**, daß
der pH-Wert der auf die keratinischen Fasern aufgetragenen Zusammensetzung 3 bis 6,9 beträgt.

11. Verfahren gemäß jedem der Ansprüche 1 bis 10,
dadurch **gekennzeichnet**, daß
die zur Färbung der keratinischen Fasern verwendete Zusammensetzung zusätzlich zu den heterozyklischen Kupplern der Familie der 5-, 6- oder 7-Hydroxyindole der Formel (I) weitere Kuppler enthält, ausgewählt aus m-Diphenolen, m-Aminophenolen, m-Phenylendiaminen, m-Acylaminophenolen, m-Ureidophenolen, m-Carbalkoxyaminophenolen, α-Naphthol, Kupplern mit einer aktiven Methylengruppe, wie β-Ketoverbindungen und Pyrazolonen.

12. Verfahren gemäß Anspruch 11,
dadurch **gekennzeichnet**, daß
die Kuppler aus 2,4-Dihydroxyphenoxyethanol, 2,4-Dihydroxyanisol, m-Aminophenol, Resorcin, dem Monomethylether von Resorcin, 2-Methylresorcin, 2-Methyl-5-N-(β-hydroxyethyl)aminophenol, 2-Methyl-5-N-(β-mesylaminoethyl)aminophenol, 6-Hydroxybenzomorpholin, 2,4-Diaminoanisol, 2,4-Diaminophenoxyethanol, 6-Aminobenzomorpholin, (2-N-(β-Hydroxyethyl)amino-4-amino)phenoxyethanol, 2-Amino-4-N-(β-hydroxyethyl)aminoanisol, (2,4-Diamino)phenyl-β,γ-dihydroxypropylether, 2,4-Diaminophenoxyethylamin, 2-Methyl-5-aminophenol, 2,6-Dimethyl-3-aminophenol, 3,4-Methylendioxyphenol, 3,4-Methylendioxyanilin sowie deren Salzen ausgewählt sind.

13. Verfahren gemäß jedem der Ansprüche 1 bis 12,
dadurch **gekennzeichnet**, daß
die Zusammensetzung anionische, kationische, nicht-ionische, amphotere oberflächenaktive Mittel oder deren Mischungen, Verdickungsmittel, Antioxidantien und/oder jeden weiteren kosmetisch verträglichen Hilfsstoff enthält.

14. Verfahren gemäß jedem der Ansprüche 1 bis 13,
dadurch **gekennzeichnet**, daß
das zur Färbung geeignete Milieu aus Wasser oder einer Mischung aus Wasser und einem Lösungsmittel zusammengesetzt ist, ausgewählt aus C₂₋₄-Niedrigalkanolen, Glycerin, Glycolen oder Glycolethern, dem Monoethylether und Monomethylether von Diethylenglycol, aus aromatischen Alkoholen oder Phenoxyethanol oder aus deren Mischungen.

15. Mittel zur Färbung keratinischer Fasern und insbesondere der Haare,
dadurch **gekennzeichnet**, daß
es mindestens zwei Bestandteile enthält, wobei ein Bestandteil (A) aus einer Zusammensetzung, die in einem zur Färbung geeigneten Milieu einen in jedem der Ansprüche 1 bis 3 definierten indolischen Kuppler der Formel (I) und eine in jedem der Ansprüche 1 und 4 bis 12 definierte Oxidationsfarbstoff-Vorstufe enthält, und ein Bestandteil (B) aus einer Zusammensetzung zusammengesetzt sind, die in einem zur Färbung geeigneten Milieu ein Oxidationsmittel enthält, wobei der pH der Bestandteile (A) und (B) bei einem solchen Wert liegt, daß nach Vermischung in Mengenverhältnissen von 90 bis 10% für den Bestandteil (A) und von 10 bis 90% für den Bestandteil (B) die sich ergebende Zusammensetzung einen pH unterhalb 7 aufweist.

16. Mittel gemäß Anspruch 15,
dadurch **gekennzeichnet**, daß
der Bestandteil (A) einen pH-Wert von 3 bis 10,5 hat.

17. Mittel gemäß Anspruch 15 oder 16,
dadurch **gekennzeichne**t, daß
der Bestandteil (A) die Oxidationsfarbstoff-Vorstufen vom para- und/oder ortho-Typ sowie die Kuppler in Mengenverhältnissen von 0,3 bis 7 Gew.%, bezogen auf das Gesamtgewicht des Bestandteils (A), enthält.

18. Mittel gemäß jedem der Ansprüche 15 bis 17,
dadurch **gekennzeichnet**, daß
die Konzentration an Verbindungen der Formel (I) 0,05 bis 4 Gew.%, bezogen auf das Gesamtgewicht des Bestandteils (A), beträgt.

19. Mittel gemäß jedem der Ansprüche 15 bis 18,
dadurch **gekennzeichne**t, daß
der Bestandteil (A) oberflächenaktive Mittel in Mengenverhältnissen von 0,1 bis 55 Gew.%, Losungsmittel zusätzlich zum Wasser in Mengenverhältnissen von 0,5 bis 40 Gew.%, Verdickungsmittel in Mengenverhältnissen von 0,1 bis 5 Gew.%, Antioxidantien in Mengenverhältnissen von 0,02 bis 1,5 Gew.% und/oder jeden weiteren kosmetisch verträglichen Hilfstoff enthält.

20. Mittel gemäß jedem der Ansprüche 15 bis 19,
dadurch **gekennzeichnet**, daß
der Bestandteil (B) einen pH mit einem Minimalwert von 1 und unterhalb 7 aufweist.

21. Verfahren zur Färbung keratinischer Fasern und insbesondere der Haare,
dadurch **gekennzeichnet**, daß
man in einer ersten Stufe ein in jedem der Ansprüche 15 bis 20 definiertes Färbemittel vorhält und vor der Aufbringung die Vermischung der Bestandteile (A) und (B) in Mengenverhältnissen von 10 bis 90% für den Bestandteil (A) und von 90 bis 10% für den Bestandteil (B) durchführt, und zwar so, daß eine Zusammensetzung mit einem pH unterhalb 7 erhalten wird, und daß man diese Mischung unmittelbar nach der Zubereitung auf die keratinischen Fasern aufträgt.

22. Vorrichtung aus mehreren Teilen oder Kit zur Färbung,
dadurch **gekennzeichnet**, daß
sie mindestens zwei Teile umfassen, wovon ein erstes Teilstück den in jedem der Ansprüche 15 bis 20 definierten Bestandteil (A) und das zweite Teilstück den in den Ansprüchen 15 bis 20 definierten Bestandteil (B) enthalten.

23. Vorrichtung gemäß Anspruch 22,
dadurch **gekennzeichnet**, daß
sie mit einem Element ausgerüstet ist, das es ermöglicht, auf die Haare die erwünschte Mischung aus den Bestandteilen (A) und (B) auszubringen und aufzutragen.

24. Färbeverfahren gemäß jedem der Ansprüche 1 bis 14,
dadurch **gekennzeichnet**, daß
man auf die Haare die Zusammensetzung aufträgt und sie 3 bis 40 Minuten lang verweilen läßt, die Haare spült und vor einer erneuten Spülung und Trocknung gegebenenfalls schamponiert.

25. Verwendung der in jedem der Ansprüche 1 bis 3 definierten 5-, 6- oder 7-Hydroxyindolderivate der Formel (I) als Kuppler zur Färbung keratinischer Fasern in saurem Milieu, zusammen mit Oxidationsfarbstoff-Vorstufen.

26. Zusammensetzung zur Färbung keratinischer Fasern zur gebrauchsfertigen Anwendung in dem Verfahren gemäß jedem der Ansprüche 1 bis 14.

27. Zusammensetzung gemäß Anspruch 26, welche die Verbindung der Formel (I) in Mengenverhältnissen von 0,01 bis 3,5 Gew.%, bezogen auf das Gesamtgewicht der Zusammensetzung, enthält.
